# EUROPEAN PATENT APPLICATION

(11) **EP 1 488 750 A1**
(43) Date of publication of application: **22.12.2004**
(21) Application number: 04474001.7
(22) Date of filing: 17.06.2004
(51) Int. Cl.: A61B 17/64

(54) **Pelvis Tongs**

(30) Priority: 18.06.2003 SK 7752003
(71) Applicant: Briancin, Jaroslav, 040 22 Kosice (SK)
(72) Inventor: Briancin, Jaroslav, 040 22 Kosice (SK)

(57) **Abstract**

The present invention is pelvis tongs witch composes of two parts. The main supported curved hollow holder (1) with locked screw (6) and in this holder is putting little shoulder (5). At the second end of little shoulder (5) is attached fixing mechanism (4). The second end of the main supported curved hollow holder is narrowed to size and shape its own hollow inner diameter, what is created overextended operating part (2). Instrumental curved hollow holder (7) with fast securing joint (3) is sliding putting in the overextended operating part (2). The shoulder (5) with fixing mechanism (4) is putting in the other side of instrumental angled hollow holder (7). The overextended operating parts of holder (2) are pushing at the one end of main supported curved hollow holder (1) tightly and/or take apart.

## Description

### FIELD OF THE INVENTION

The present invention relates pelvis tongs used for pressing stabilisation of dislocated pelvis ring fracture of unstable patient in hard haemorrhage trauma or for pressing pelvis reposition and pelvis immobilisation. Pelvis tongs comprises pair of main supported curved hollow holder with overextended operating part. And parenthetical in this is sliding instrumental bent hollow holder with fast custodial joint. Besides all free ends of main supported curved hollow holder and instrumental angled hollow holder with locked screw are attached to little shoulders with fixing mechanism.

### BACKGROUND OF THE INVENTION

There are known pelvis tongs, made by trademark "Fiving", and comprises of sliding rectangular frame. On this frame, there are putting on two sideways shoulders with lock and on both free ends of shoulders, there are placed the barbs with screw mechanism. AO tongs are used for few years. As technical modification of these tongs works ACE tongs, which are similar with enlarged medical tongs. Surgery procedures are same by using both of tongs. Correct axial place of clip supply tongs AO, which are made on principle of joiner's tongs. Opposite, is very hard to make correct axial place with ACE tongs, which has three joints for mobility of clips. Medical research was showed ACE tongs are usually used. Literature summary showed that there exist any comprehensible instructions how the best to operate injured patients by moving pelvis ring. Because that, there was discovering pelvis C-tongs. Pelvis tongs works as carpenter's vice and they used diagonal pressure right over SI joint. They comprised of two supports at both sides, which are moving on holder. On the free end of each support is an opening, in what is placed thread tubule with inner nail. A nail from both sides is installed to bone by stabbing cut, in this way, that the supports are sliding towards each other. Spike of nail is beating in pelvis bone. If it is necessary, then there is applicated Steiman clip, placed over tip of pelvis bone for last manipulation, in the point of traction on leg. Thread tubule is secured and pelvis ring is pressed and tightly fixed. Experiment shows that average coercively pressuring to location of SI joint is 342 N. Tongs are applicated on patient in position lying on back. There are used no special device, if it necessary can be used local anaesthesia in trauma room or on rtg table. ACE tongs was designed according to medical tongs with spikes, but in bigger size. The tongs consist of three joints; central joint works as rotary point of curved sideways shoulders. A pawl in rotary point is locking main pressure, immediately when sideways shoulders are bringing closer toward you. Another two joints are placed in point of tongs junction with sideways shoulder and helps to secure right axial location. The compression by pressure on both sides of shoulders can be gradually left over by screw tightening on tongs, sideways shoulder like on C-tongs. ACE tongs are made of titanium and weigh 2,7 kg. Another known is AO/ASIF pelvis C - tongs. AO pelvis C - tongs works similar like joiner's tongs. Sideways shoulders fix the clips with the ends fitting tightly on a bone to holder leading parallel to the clips. Sideways shoulders can easy move on this leading holder. The compression of sideways shoulders bring about compression of pelvis ring, or SI tight bone joint bringing on fractured surface. This way running compression depends on fixation of sideways shoulders on leading holder.

Disadvantage of present and now used pelvis tongs is in: they are constructional complicated, they have many mechanical parts, witch needs setting and securing and surgeon has hard work. Each mechanical part is higher weight of pelvis tongs and more operoseness for pressing stabilisation of dislocated pelvis ring fracture of unstable patient in hard haemorrhage trauma or for pressing pelvis reposition and pelvis immobilisation. For high solidity of now used pelvis tongs they are made of special steel and alloy. These expensive materials do high production cost.

### SUMARY OF THE INVENTION

Said disadvantages, to a large degree, eliminate pelvis tongs, made of main supported bent hollow holder with locked screw. There is a shoulder with fixing mechanism at one side of the holder and at the other side is narrowed down to size and shape of hollow inner diameter of the holder. This way is made long operating part of holder, where is sliding instrumental curved hollow holder with fast custodial joint at the one end. Instrumental bent hollow holder has putting on little shoulder with fixing mechanism at the second end.

There is putting in little shoulder with fixing mechanism at the one side of the main supported bent hollow holder with locked screw and the second side comprises of tight or take apart long operated part of holder.

Pelvis tongs according to patent allows fast aretation of sliding instrumental bent hollow holder with main supported angled holder by fast securing joint.

A new design and shape of pelvis tongs allows problemless stabilisation of dislocated pelvis ring fracture or pelvis reposition and pelvis immobilisation also large patients not just in orthogonal location, but also in variously bevelled locations around axis between spikes of fixing mechanism of pelvis tongs.

Pelvis tongs according of invention advantage is in this, that overextended operating part of main supported curved hollow holder delimit required distance between the spikes of both fixing mechanisms.

Another present invention's advantage is: there is unnecessary using any instrumental tool or spanner to setting or securing of pelvis tongs, because of fast securing joint. Surgeon manually sets and secures pelvis tongs, what is very sensitive consolidation of pelvis tongs on the patient's body. This way reduces risk of damage of part of pelvis by supported spikes in contact points.

High stability of pelvis tong's framework allows fastening also the largest patients without using instrumental addition or another types of tongs.

There is no necessary to use special expensive steel.

### BRIEF DESCRIPTIONS OF THE DRAWINGS

The present invention is described in detail bellow with reference to the drawings in which:
FIG. 1 is a cross-section perspective of the pelvis tongs of the present invention;

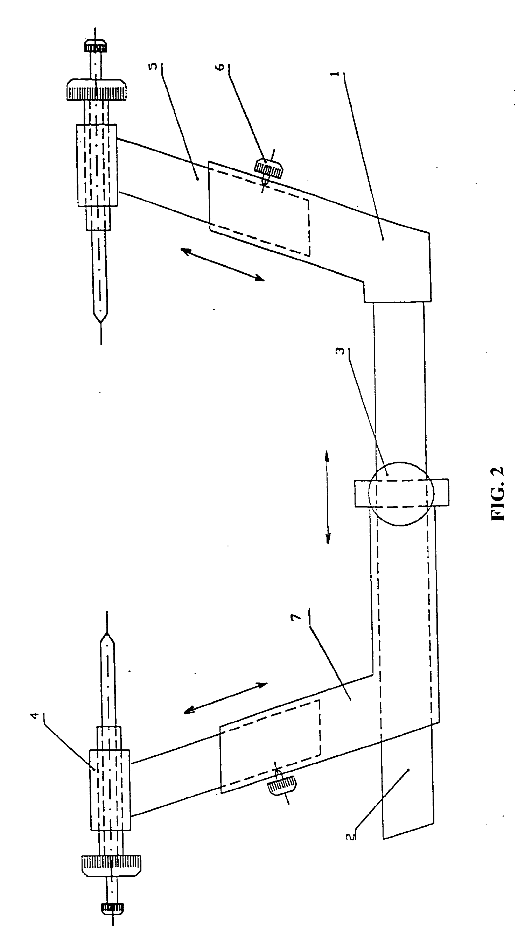

FIG. 2 is a perspective of an illustrative embodiment of all directions of motion of the moving parts of pelvis tongs.

### DETAILED DESCRIPTION OF THE INVENTION

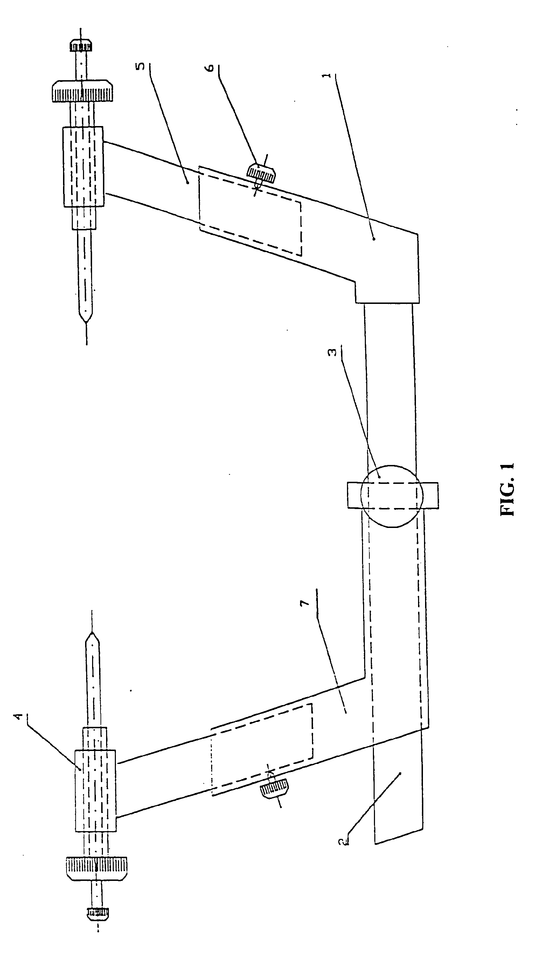

The pelvis tongs according FIG. 1 comprises of main supported to the shape for example slanting inverse letter "L" curved hollow holder 1. There is placed locked screw 6 on main supported bend hollow holder 1 for setting and securing of tongs height by sliding of little shoulder 5 to the body of main supported angled hollow holder 1. There is attached fixing mechanism 4 with spike on the outer end of little shoulder 5. The second end of main supported curved hollow holder 1 is narrowed on size and shape of hollow inner diameter of the holder, what is overextended operating part 2, on which is putting on instrumental hollow holder 7. This sliding instrumental holder 7 is bending to the shape for example slanting inverse letter "L". There is located fast securing joint 3 for setting and securing of pelvis tongs extension on the one end of instrumental curved hollow holder 7. The little shoulder 5 is putting on the second end of the instrumental curved hollow holder 7. There is by the opposing spikes attached fixing mechanism 4 on the free end of little shoulder 5. The required distance between spikes of both of fixing mechanisms 4 is defined by moving of instrumental curved hollow holder 7 along overextended operating part of holder 2 of main bent hollow holder 1.

The pelvis tongs are securing by fast-secured joint 3 after putting and pressing of main curved holder 1 with instrumental bent hollow holder 7. The tips are putting in pelvis bone by pelvis tongs and fixing mechanisms 4. In this way the pelvis ring is compressed and the fix joint of two bones is completed. This compression depends on fixing of instrumental curved hollow holder 7 on overextended operating part of holder 2 of main curved hollow holder 1.

The main supported bent hollow holder 1 with locked screw 6 according FIG. 1 has sliding little shoulder 5 with fixing mechanism 4 at one side. And at the other side has created fix or take apart long operated part of holder 2.

## Claims

1. The pelvis tongs comprising of a pair of sideways shoulders jointed by rotating joint at the one end and having the clips at the free ends used for pressing stabilisation of dislocated pelvis ring fracture of unstable patient in hard haemorrhage trauma or for pressing pelvis reposition and pelvis immobilisation is **characterised in that** it is composed of main supported curved hollow holder (1) with locked screw (6), in witch is putting little shoulder (5) with fixing mechanism (4) from the one side and its second end is narrowed on the size and shape its own hollow inner diameter, witch is creating overextended operating part of holder (2), in what is putting sliding instrumental bent hollow holder (7) with fast securing joint (3) at one side and at the other side is putting shoulder (5) with fixated mechanism (4).

2. The pelvis tongs according to claim 1, **characterised in that** there is putting in little shoulder (5) with fixing mechanism (4) at the one side of the main supported bent hollow holder (1) with locked screw (6) and the second side comprises of fix overextended operated part of holder (2).

3. The pelvis tongs according to claim 2, **characterised in that** there is putting in little shoulder (5) with fixing mechanism (4) at the one side of the main supported bent hollow holder (1) with locked screw (6) and the second side comprises of take apart overextended operated part of holder (2).
